(19) **Europäisches Patentamt**

**European Patent Office**

**Office européen des brevets**

(11) **EP 0 200 344 B1**

(12) **EUROPEAN PATENT SPECIFICATION**

(45) Date of publication and mention
of the grant of the patent:
**11.11.1998 Bulletin 1998/46**

(51) Int. Cl.$^6$: **A01N 63/00**, C12N 15/00

(21) Application number: **86302122.6**

(22) Date of filing: **21.03.1986**

(54) **Microorganisms as pesticides**

Mikroorganismen als Pestizide

Microorganismes comme pesticides

(84) Designated Contracting States:
**BE CH DE FR GB IT LI NL SE**

(30) Priority: **28.03.1985 US 717207**
**09.09.1985 US 774120**
**05.02.1986 US 826404**

(43) Date of publication of application:
**10.12.1986 Bulletin 1986/45**

(73) Proprietor: **MYCOGEN CORPORATION**
**San Diego, California 92121 (US)**

(72) Inventors:
• **Edwards, David L.**
**Del Mar California 92014 (US)**
• **Rammler, David H.**
**Woodside California 94062 (US)**
• **Gaertner, Frank H.**
**San Diego California 92109 (US)**

(74) Representative:
**Perry, Robert Edward et al**
**GILL JENNINGS & EVERY**
**Broadgate House**
**7 Eldon Street**
**London EC2M 7LH (GB)**

(56) References cited:
EP-A- 0 164 245        EP-A- 0 192 319
EP-A- 0 209 370        EP-A- 0 213 818
EP-A- 0 228 838        WO-A-87/03303

• **ENGINEERED ORGANISMS IN THE
ENVIRONMENT, 1985, pages 40-46, American
Society for Microbiology, Washington, US; L.S.
WATRUD et al.: "Cloning of the bacillus
thuringiensis subsp. kurstaki delta-endotoxin
gene into pseudomonas fluorescens: molecular
biology and ecology of an engineered microbial
pesticide"**
• **CHEMICAL ABSTRACTS, vol. 105, no. 25, 22nd
December 1986, pages 213-214, abstract no.
220216b, Columbus, Ohio, US; M.G.
OBUKOWICZ et al.: "Tn5-mediated integration of
the delta-endotoxin gene from bacillus
thuringiensis into the chromosome of root-
colonizing pseudomonads", & J. BACTERIOL.
1986, 168(2) 982-9**
• **CHEMICAL ABSTRACTS, vol. 106, no. 15, 13th
April 1987, page 231, abstract no. 115276u,
Columbus, Ohio, US; & JP-A-61 239 885
(MYCOGEN CORP.) 25-10-1986**
• **GENETIC ENGINEERING NEWS, vol. 5, no. 2,
February 1985, pages 1,8; M.A. LIEBERT, Inc.; J.
STERLING: "Monsanto to perform first bio-
pesticide field test"**

## Description

This invention relates to the use of microorganisms as pesticides.

A number of naturally-occurring microorganisms produce polypeptides which are capable of controlling the proliferation of many agricultural pests. For a number of reasons, these naturally-produced pesticides have not found widespread commercial exploitation, despite their ecological desirability. In many cases, the relevant organism is difficult to grow, the cost of the pesticide is high, and the residual activity of the pesticide in the environment is relatively low. In individual instances, there are additional drawbacks.

Microorganisms, like most living things, occupy specialised niches in the environment. A comprehensive review, entitled "Ecology and Epidemiology of Foliar Bacterial Plant Pathogens", is by Hirano et al, Annual Review of Phytopathology 21 (1983) 243-69. Survivability is usually adversely affected when an organism is introduced into an exotic environment. Such is the case for naturally-occurring microorganisms that produce pesticides. In order to be effective as pesticides, the naturally-occurring microorganisms and/or their products must be introduced into exotic environments and, therefore, have insufficient residual activity. However, the potency of the pesticides and their ecological desirability make them attractive candidates for commercial exploitation, if the disadvantages associated with their use could be removed or at least ameliorated.

There is, therefore, substantial interest in finding economical ways to produce these pesticides, to deliver them to the field in a bioactive form, and to enhance and prolong their activity in the environment.

Bulla et al, (1981) J. Biol. Chem. 254:3000-3004, describe the proteinaceous parasporal crystal toxin fro B. thuringiensis (Bt) var. kurstaki. Held et al, (1982) Proc. Natl. Acad. Sci. USA 79:6065-6069, describe the cloning of the gene for proteinaceous parasporal crystal toxin into E. coli and B. subtilis. See also Schnepf et al, ibid (1981) 78:289-294. Wong et al, (1983) J. Biol. Chem. 258:1960-1967, describe the transcriptional regulatory signals of the Bt toxin gene. Klier et al, (1982) EMBO 1:791-799, describe the cloning and expression of a Bt toxin gene. US-A-4265880 describes embedding live insecticidal pathogens in a coacervate microbead.

EP-A-0192319 describes biological pesticides encapsulated in non-proliferating cells. For example, a pesticide is produced by expression of a heterologous gene in a suitable host. The cells are killed under conditions which prolong the pesticidal activity when applied to the environment, e.g. lettuce plants, of a target pest.

EP-A-0213818 describes a Bt strain which produces an intracellular toxin by expression, and which can be administered to the environment of a susceptible pest.

According to the present invention, vegetation other than lettuces is protected from a eukaryotic pest by administering to the phylloplane in the field a live microorganism containing a heterologous gene which is expressed in the microorganism and which codes for a proteinaceous parasporal crystal toxin of B. thuringiensis, e.g. var. kurstaki, or B. sphaericus, wherein the live microorganism is capable of proliferation upon the phylloplane, and is a species of Pseudomonas or Rhodotorula.

The microorganisms used in the invention are produced by modifying organisms capable of occupying, surviving and proliferating in the phytosphere of plants. The microorganisms are modified by introduction of one or more genes capable of expression of the toxin in the microorganism. The preferred microorganisms grow with the plant, continually produce pesticide and protect the pesticide from environmental degradation. These microorganisms (biological packages) serve to deliver the toxin to, and maintain its activity in, the phytosphere of plants.

Microorganisms are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation. Suitable bacterial species are Pseudomonas syringae and Pseudomonas fkyirescebs; suitable yeast species are Rhodotorula rubra and R. glutinis. Such microorganisms are readily available from natural sources or established culture repositories, such as NRRL and ATCC.

Among naturally-occurring toxins are the polypeptide crystal toxins of B. thuringiensis var. kurstaki, active against lepidoptera; B. t. var. israelensis, active against mosquitoes; B. t. M-7, active against coleoptera; and B. sphaericus, active against mosquito larvae. Cultures exemplifying the above are as follows:

Bacillus thuringiensis var. kurstaki HD-1, NRRL B-3792 (disclosed in US-A-4,448,885)
Bacillus thuringiensis var. israelensis, ATCC 35646
Bacillus thuringiensis M-7, NRRL B-15939

The following B. thuringiensis cultures are available from the United States Department of Agriculture (USDA) at Brownsville, Texas, USA:

B. thuringiensis HD2

B. thuringiensis var. finitimus HD3
B. thuringiensis var. alesti HD4
B. thuringiensis var. kurstaki HD73
B. thuringiensis var. sotto HD770
B. thuringiensis var. dendrolimus HD7
B. thuringiensis var. kenyac HD5
B. thuringiensis var. galleriae HD29
B. thuringiensis var. canadensis HD224
B. thuringiensis var. entomocidus HD9
B. thuringiensis var. subtoxicus HD109
B. thuringiensis var. aizawai HD11
B. thuringiensis var. morrisoni HD12
B. thuringiensis var. ostriniae HD501
B. thuringiensis var. tolworthi HD537
B. thuringiensis var. darmstadiensis HD146
B. thuringiensis var. toumanoffi HD201
B. thuringiensis var. kyushuensis HD541
B. thuringiensis var. thompsoni HD542
B. thuringiensis var. pakistani HD395
B. thuringiensis var. israelensis HD567
B. thuringiensis var. indiana HD521
B. thuringiensis var. dakota
B. thuringiensis var. tohokuensis HD866
B. thuringiensis var. kumanotoensis HD867
B. thuringiensis var. tochigiensis HD868
B. thuringiensis var. colmeri HD847
B. thuringiensis var. wuhanensis HD525
Bacillus cereus--ATCC 21281
Bacillus moritai--ATCC 21282
Bacillus popilliae--ATCC 14706
Bacillus lentimorbus--ATCC 14707
Bacillus sphaericus--ATCC 33203
Beauveria bassiana--ATCC 9835
Metarrhizium anisopliae--ATCC 24398
Metarrhizium flavoviride--ATCC 32969
Streptomyces avermitilus--ATCC 31267

The toxin need not be the same as a naturally occurring toxin. Polypeptide toxins may be fragments of a naturally-occurring toxin; expression products of deletion, transversion or transition mutations, where two or fewer number percent of the amino acids may be changed; or a repetitive sequence capable of processing by the intended pest host. In addition, fusion products may be prepared where one, five or more amino-acids are provided at the N-terminus to provide, for example, reduced proteolytic degradation of the toxin(s). In some instances, a plurality of the same or different toxins may be encoded and expressed, where processing sites may be introduced between each toxin moiety in the polytoxin.

A wide variety of ways are available for introducing the gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will occur only after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop codon(s), a terminator region, and optionally, a polyadenylation signal.

In the direction of transcription, namely in the 5' to 3' direction of the coding or sense sequence, the construct will involve the transcriptional regulatory region, if any, and the promoter, where the regulatory region may be either 5' or 3' of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for tranformation of a microorganism host, but will usually be included with a DNA sequence involving a marker, where the second DNA sequence may be joined to the toxin expression construct or may be combined as a separate DNA fragment with the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototrophy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin producing host, so that it may effectively compete with the wild-type microorganisms and stably occupy a niche in the environment of the vegetation to be protected.

Where no functional replication system is present, the construct will also include a sequence of at least 50 bp, preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that the toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898; 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069 and Bagdasarian et al., (1981) Gene 16:237, and U.S. Patent Nos. 4,356,270;4,362,817 and 4,371,625.

The structural gene expressing the toxin may be isolated from its host in a variety of ways. Where the structural gene has been described in the literature, and sequenced and/or restriction mapped, and a sequence is available for preparing a probe, various techniques exist for preparing a library, determining which clone(s) in the library contains a complementary sequence to the probe, isolating the DNA fragment containing the desired structural gene and then modifying the fragment, as appropriate. Modification may include complete or partial digestion with one or more restriction enzymes, resection, primer repair, in vitro mutagenesis, or the like, where the entire structural gene, or a fragment having toxin activity is isolated.

The structural gene may then be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

While several polypeptide toxins are known which are heterologous to the ultimate host (by heterologous is intended products not normally produced by the ultimate host), the preferred polypeptide toxins are related to the naturally-occurring proteinaceous parasporal crystal toxins produced by wild-type B. thuringiensis. One or more structural genes encoding for different polypeptide toxins may be introduced into a host, either on the same or different compatible plasmids or under conditions of integration into the host genome.

The toxins produced in the heterologous host need not be identical to the toxin as prepared by its native host, even where it shares the same polypeptide sequence, since the toxin may be subject to varying degrees of glycosylation, depending upon the particular host.

The transformants may be isolated in accordance with conventional ways, usually employing a selection technique, which allows for selection of the desired organism as against unmodified organisms or transferring organisms, when present. The transformants may then be tested for pesticidal activity, particularly insecticidal activity. Various insect pests include diptera (flies and mosquitoes), lepidoptera (moths and butterfly larvae), coleoptera (beetle larvae and adults), and the like. For the assay of insecticidal activity the larvae of the tobacco hornworm, Manduca sexta, or cabbage looper, Trichoplusia ni may be employed according to the procedures described by Schnepf et al., supra.

Preferred hosts, particularly those in the phytosphere, will have certain characteristics which enhance the environmental stability of the toxins in the host. Protective qualities include a low level of proteolytic degradation, thick cell walls, pigmentation, and the like. Other characteristics of interest for the host include leaf affinity, lack of mammalian toxicity, attractiveness to pests for ingestion, ease of handling and storage, rate of proliferation in the field, competitiveness, and the like.

In the field applications, the transformant strain will be applied to its natural habitat, such as the rhizosphere or phylloplane of the plant to be protected from the pest. The transformant strain will grow in its natural habitat, while producing the toxin which will be absorbed and/or ingested by the larvae or adult pest, or have a toxic effect on the ova. The persistence of the microorganisms will provide for long-term protection of the vegetation, although repetitive administrations may be required from time to time. The organism may be applied by spraying, soaking, injection into the soil, seed coating, seedling coating or spraying, or the like. Where administered in the field, generally concentrations of the organism will be from $10^6$ to $10^{10}$ cells/ml, and the volume applied per hectare will be generally from 3 to 900 g, or more. Where administered to a plant part, the concentration of the organism will usually be from $10^3$ to $10^6$ cells/cm$^2$.

In aquatic environments, pesticidal control may be attained below the surface by varying the lipid content of the transformant microorganism strain. It is known that indigenous aquatic algae float due to their lipid content. A variation in lipid content will allow the transformant strain to be distributed at desired depths below the water surface.

For commercial formulations, the organisms may be maintained in a nutrient medium which maintains selectivity and results in a low rate of proliferation. Various media may be used, such as yeast extract or L-broth. Once the organism is to be used in the field, the non-proliferating concentrate may be introduced into a appropriate selective nutrient medium, grown to high concentration, generally from about $10^5$ to $10^9$ cells/ml and may then be employed for introduction into the phylloplane or rhizosphere or for treatment of a plant part, e.g., seed, tuber, and the like; seedling treatment or the like.

The following examples are offered by way of illustration and not by way of limitation.

Example 1--Construction of A Heterologous Gene and Transformation into A Suitable Host.

A construction began with a clone of Pseudomonas aeruginosa, available from Northern Regional Research Laboratories (NRRL B-12127), containing a broad host range shuttle plasmid pRO1614 (J. Bact. [1982] 150:60; U.S. Patent No. 4,374,200). The plasmid has unique HindIII, BamHI, and SalI and PvuII restriction sites, a PstI insertion, which includes the carbenicillin resistance gene and a P. aeruginosa replication system, where the HindIII, BamHi and SalI restriction sites are in a tetracycline resistance gene. The remainder of the plasmid is derived from pBR322. A second plasmid, pSM1-17, has been deposited as a clone of E. coli (NRRL B-15976). This deposit was made with the permanent collection of the Northern Regional Research Laboratory, U.S. Department of Agriculture, Peoria, Illinois 61604, USA. Plasmid pSM1-17 confers ampicillin resistance to E. coli and contains a 6.8 Kbp HindIII DNA fragment that includes the δ-endotoxin gene from the 50 md plasmid of Bacillus thuringiensis HD73. Sufficient toxin is expressed from this gene in E. coli to make the intact cells toxic to cabbage loopers. A further modification of the DNA fragment was done to enhance toxin expression and to accomplish expression of toxin in an alternative host, Pseudomonas fluorescens. In order to eliminate unwanted DNA from the 6.8 Kbp fragment, pSM1-17 was digested with BamH1 to open the circular plasmid at a site nearest to the 5' end of the toxin gene, and was treated with the exonuclease Bal31 to reduce the 6.8 Kbp HindII insert to about 2.9 Kbp. The free ends of the DNA were filled in with Klenow polymerase, BamH1 linkers were added; and the linear DNA was ligated to reform the circular plasmid. The resultant vector, pFG270, was digested with XhoI to open the circular plasmid at a site nearest to the 3' end of the toxin gene. SalI linkers were added, the plasmid was ligated to its circular form, and the resultant vector, pFG10.6 was amplified in E. coli. pFG10.6 was digested completely with SalI and BamHI and the resulting 2.1 Kbp fragment containing the toxin gene was purified by gel electrophoresis and ligated into the BamHI and SalI sites of plasmid pRO1614. The resultant plasmid, pCH2.1, was amplified in E. coli and used to transform Pseudomonas fluorescens to carbenicillin resistance and the ability to synthesize δ-endotoxin. Pseudomonas fluorescens (pCH2.1) was deposited with NRRL and was given the accession number NRRL B-15977.

The culture deposits E. coli(pSM1-17), NRRL B-15976 and P. fluorescens(pCH2.1), NRRL B-15977, were made on 2nd July 1985; they are in the permanent collection of the NRRL repository.

In the following illustrative experiments, P. fluorescens (pCH2.1) was used in conjunction with controls of untransformed cells.

Example 2 Testing of Microbes Hosting A Heterologous Gene

Two Pseudomonas fluorescens strains which had been transformed with plasmid pCH2.1, were used. These strains were used in conjunction with controls of untransformed cells.

Newly-hatched cabbage loopers are placed in a Petri dish containing droplets of the material to be bioassayed. The larvae imbibe the liquid and are then removed to small cups containing larval diet. The larvae are examined after seven days and the total number of animals killed is recorded. The following Table indicates the results.

| Bioassay 1 - P. fluorescens (strain 1) | | | | |
|---|---|---|---|---|
| Microorganism | Treatment | # Larvae Killed/Total | % Larvae Killed | Viable Cell Count/ml |
| P. fluorescens | Live | 0/15 | 0 | 8 x 10E11 |
| P. fluorescens + Bt Toxin | Live | 11/18 | 62 | 2.5 x 10E12 |
| P. fluorescens | 1% Lugol's 4 hrs | 0/15 | 0 | 0 |
| P. fluorescens + Bt Toxin | 1% Lugol's 4 hrs | 8/13 | 62 | 0 |

| Bioassay 2 - P. fluorescens (strain 1) | | | | |
|---|---|---|---|---|
| Microorganism | Treatment | # Larvae Killed/Total | % Larvae Killed | Viable Cell Count/ml |
| P. fluorescens | Live | 0/15 | 0 | 3.7 x 10E11 |
| P. fluorescens + Bt Toxin | Live | 3/20 | 15 | 4.5 x 10E11 |
| P. fluorescens | 1% Lugol's 4 hrs | 0/15 | 0 | 0 |
| P. fluorescens + Bt Toxin | 1% Lugol's 4 hrs | 8/15 | 53 | 0 |

| Bioassay 3 - P. fluorescens (strain 2) | | | |
|---|---|---|---|
| Microorganism | # Larvae Killed/Total | % Larvae Killed | Viable Cell Count/ml |
| P. fluorescens | 6/14 | 30 | 3 x 10E11 |
| P. fluorescens + Bt Toxin | 20/20 | 100 | 3 x 10E10 |

Example 3--Persistence of Microorganisms on Leaf Surfaces

This example gives the evaluation of various microorganisms for the property of colonizing plant leaves. The better the capacity of the microorganism to colonize leaves, the more useful it is in the subject invention. The following results show, surprisingly, that certain yeasts, for example Rhodotorula rubra, are, advantageously, very good colonizers of plant leaves. Thus, these microorganisms are very useful in the practice of the subject invention.

Cells of the given (in the following Table) organisms were sprayed in water at about 10E8/ml. Leaves were washed under standardised conditions and samples were plated for counts and identification of microorganisms. Background counts were from 10E1 to 10E3/cm$^2$. The following Table records the normalised half-life (NHL) which is the expected time, in days, for the starting concentration of cells on the leaf surface of tomato plants to drop 50% (NHL = Log 0.5/-b , where -b is the slope of the linear regression of the decay curve; Log cells/cm$^2$ vs time). A result indicated as "500$^+$" means that cells were maintained at 10E5 to 10E6/cm$^2$ for 4 weeks or more; the calculated half-life is longer than 500 days. The assays were preformed in southern California over a nine-month period from November to July.

| Organism | Result |
|---|---|
| Pseudomonas fluorescens leaf isolate | 4.9 |
| Rhodotorula rubra | 500$^+$ |
| Rhodotorula glutinis | 8.7 |

The same test was conducted, using Rhodotorula rubra, on various other plants. The plants and the NHL values were cucumber (99), zucchini (34), cabbage (9), ash (500$^+$), birch (500$^+$), elder (500$^+$), oak (500$^+$), plum (12), poplar (7), sequoia (500$^+$), sycamore (15) and willow (17).

The above results show that Rhodotorula rubra is the best epiphytic colonizer. Bacillus thuringiensis toxins can be expressed in Rhodotorula rubra using plasmids containing a yeast promoter such as alcohol dehydrogenase (Bennetzen and Hall [1982] J. Biol. Chem. 257:3018; Ammerer, in Methods in Enzymology [1983] Vol. 101, p. 192), phosphoglycerate kinase (Derynck et al, in Experimental Manipulation of Gene Expression [1983] p. 247, ed. M. Inouye, Academic Press), triose phosphate isomerase (Alber and Kawasaki [1982] J. Molec. and Applied Genet. 1:419) or enolase (Innes et al. [1985] Science 226:21).

It is evident, from the above results, that one can introduce a structural gene encoding for a polypeptide toxin into a heterologous host, where the heterologous host is adapted for inhabiting a niche associated with a plant or other pest habitat. The heterologous host is then capable of producing the toxin and providing protection to a plant from an insect pest. This indicates that the heterologous host is ingested by the insect pest, as ingested it provides a biocidal amount of the insecticide in active form to the pest, and thus provides protection to the plant.

The subject invention provides a substantial improvement in the utilization of polypeptide toxins, in that the toxins can be introduced into heterologous hosts, which can be economically grown, can be disseminated among vegetation, both on the leaves and in the soil, and will multiply so as to provide the toxin for extended periods of time and in substantially greater amount in the field than originally produced in a manufacturing facility. The host may in addition provide protection to the toxin from environmental deterioration, provide a continuing supply of the toxin, and provide the toxin in a useful biocidal form.

## Claims

1. A method for protecting vegetation other than lettuces from a eukaryotic pest, which comprises administering to the phylloplane in the field a live microorganism containing a heterologous gene which is expressed in the microorganism and which codes for a proteinaceous parasporal crystal toxin of B. thuringiensis or B. sphaericus, wherein the live microorganism is capable of proliferation upon the phylloplane, and is a species of Pseudomonas or Rhodotorula.

2. A method according to claim 1, in which the microorganism is Pseudomonas fluorescens.

3. A method according to claim 1, in which the microorganism is Rhodotorula rubra or Rhodotorula glutinis.

4. A method according to any preceding claim, in which the toxin is of B. thuringiensis var. kurstaki.

## Patentansprüche

1. Verfahren zum Schutz von anderen Pflanzen als Salatpflanzen vor einem eukaryotischen Schädling, welches umfaßt, daß auf der Phyllooberfläche im Freiland ein lebender Mikroorganismus ausgebracht wird, der ein heterologes Gen enthält, welches in dem Mikroorganismus exprimiert wird und für ein kristallines Parasporen-Proteintoxin von B. thuringiensis oder B. sphaericus kodiert, wobei der lebende Mikroorganismus zur Vermehrung auf der Phyllooberfläche imstande und eine Spezies von <u>Pseudomonas</u> oder <u>Rhodotorula</u> ist.

2. Verfahren nach Anspruch 1, worin der Mikroorganismus <u>Pseudomonas</u> <u>fluorescens</u> ist.

3. Verfahren nach Anspruch 1, worin der Mikroorganismus <u>Rhodotorula</u> <u>rubra</u> oder <u>Rhodotorula</u> <u>glutinis</u> ist.

4. Verfahren nach irgendeinem der vorhergehenden Ansprüche, worin das Toxin von <u>B.</u> <u>thuringiensis</u> var. <u>kurstaki</u> stammt.

**Revendications**

1. Procédé pour protéger la végétation autre que les laitues contre un nuisible eucaryote, qui comprend l'administration au phylloplan dans les champs d'un micro-organisme vivant contenant un gène hétérologue qui est exprimé dans le micro-organisme et qui code une toxine cristallisée parasporale protéique de <u>B.</u> <u>thuringiensis</u> ou de <u>B.</u> <u>sphaericus</u>, où le micro-organisme vivant est capable de proliférer sur le phylloplan et est une espèce de <u>Pseudomonas</u> ou de <u>Rhodotorula</u>.

2. Procédé selon la revendication 1, où le micro-organisme est <u>Pseudomonas</u> <u>fluorescens</u>.

3. Procédé selon la revendication 1, où le micro-organisme est <u>Rhodotorula</u> <u>rubra</u> ou <u>Rhodotorula</u> <u>glutinis</u>.

4. procédé selon l'une quelconque des revendications précédentes, où la toxine est de <u>B.</u> <u>thuringiensis</u> var. <u>kurstaki</u>.